# EUROPÄISCHE PATENTSCHRIFT

(11) **EP 1 447 054 B1**
(45) Veröffentlichungstag und Bekanntmachung des Hinweises auf die Patenterteilung: **30.08.2006**
(21) Anmeldenummer: 03003102.5
(22) Anmeldetag: 12.02.2003
(51) Int. Cl.: A61B 17/92, A61B 17/72

(54) **Vorrichtung zur Implantatentfernung**
Device for the removal of implants
Instrument servant au retrait d'implants

(43) Veröffentlichungstag der Anmeldung: 18.08.2004
(73) Patentinhaber: Zimmer GmbH, 8404 Winterthur (CH)
(72) Erfinder: Gerngross, Heinz, Prof.Dr.med., 89075 Ulm (DE); Bartsch, Eric, 6415 Arth (CH)
(74) Vertreter: Manitz, Finsterwald & Partner GbR

(56) Entgegenhaltungen:
- CH-A- 301 236
- DE-C- 874 637
- US-A- 3 334 624
- US-A- 4 423 721
- US-A- 4 875 474

## Beschreibung

Die Erfindung betrifft eine Vorrichtung zur Implantatentfernung mit den Merkmalen des Oberbegriffs des Anspruchs 1.

Aus US 3,334,624 (Basis für den Oberbegriff des Anspruchs 1) ist ein Instrument bekannt, mit dem Marknägel nicht um eingeseht, sondern auch entfernt werden können. Hirzu weist das Instrument ein separates shiftförmiges Element auf, das nach dem Einsehen des Nagels von diesem gelöst und von dem Entfernen des Nagels wieder mit diesem verbunden werden muss.

Vor allem bei Operationen zur Versorgung von Knochenbrüchen werden vermehrt minimalinvasive Techniken zur Einbringung von Implantaten gefordert. Insbesondere wird diese Anforderung in Verbindung mit dem Implantieren von Marknägeln gestellt.

Bei der Implantation von Marknägeln ist man inzwischen so weit, dass lediglich noch sehr kleine Hautschnitte mit einer Länge von etwa 2 bis 3 cm benötigt werden. Problematisch ist, dass derzeit diese Minimalinvasivität nicht aufrechtzuerhalten ist, wenn nach der Heilung des Knochenbruches das Implantat wieder entfernt werden soll.

Hierzu muss das Implantat im Knochen aufgesucht und mit einem Instrumentar zum Ausschlagen verbunden werden. Dies erfordert in vielen Fällen einen wesentlich größeren Zugang zu dem Implantat als bei dessen Implantation. Vor allem aus Sicht des Patienten ist dieser Umstand schwer verständlich, denn von der eigentlichen Operation beim Einsetzen des Implantats resultierte ursprünglich lediglich eine kleine Narbe, die nun bei der Implantatentfernung massiv vergrößert werden muss.

Aufgabe der Erfindung ist es daher, eine Möglichkeit zu schaffen, die vorstehend geschilderte Problematik möglichst weitgehend zu beseitigen und sowohl beim Einsetzen als auch beim Entfernen von Implantaten mit möglichst kleinen Hautschnitten auszukommen.

Die Lösung dieser Aufgabe erfolgt durch die Merkmale des Anspruchs 1.

Erfindungsgemäß kann mittels des flexiblen Koppelorgans unabhängig davon, wo und insbesondere in welcher Tiefe sich das mit dem Implantat verbundene Verankerungselement im Körper befindet, das Verankerungselement und damit das zu entfernende Implantat problemlos aufgefunden werden. Hierzu dient der Handhabungsabschnitt der erfindungsgemäßen Entfernungsvorrichtung, der in einer für den jeweiligen Anwendungsfall geeigneten Art und Weise im Körper platziert wird.

In vorteilhafter Weise kann die Länge des Koppelorgans derart bemessen sein, dass der Handhabungsabschnitt direkt unter der Haut des Patienten platzierbar ist. Zur Implantatentfernung braucht nur mit einem sehr kleinen Schnitt die Haut an der entsprechenden Stelle inzidiert zu werden, um zu dem Handhabungsabschnitt des Koppelorgans zu gelangen. Das Koppelorgan kann dann entweder als eine Führung dienen, die den Operateur durch das Gewebe hindurch genau zu dem Verankerungselement und damit zu dem zu entfernenden Implantat führt, oder - im Fall eines vergleichsweise locker sitzenden Implantats - es kann das Implantat einfach mittels des Koppelorgans aus dem jeweiligen Knochen und aus dem Körper des Patienten herausgezogen werden.

Besonders vorteilhaft einsetzbar ist die erfindungsgemäße Implantatentfernungsvorrichtung in Verbindung mit Marknägeln, die zur Versorgung von Knochenbrüchen eingesetzt werden. Derartige Marknägel werden in der Praxis meist mit Hilfe eines Zielgerätes implantiert und weisen hierzu einen insbesondere ein Gewinde umfassenden Kopplungsabschnitt für das Zielgerät auf, der nach Abschluss der Implantation mit einem insbesondere schrauben- oder kappenartigen Abschlusselement besetzt wird, um den Kopplungsabschnitt gegen Gewebeeinwuchs zu schützen.

Erfindungsgemäß kann vorgesehen sein, dass dieses mit dem Implantat verbindbare Abschluss- oder Schutzelement als das Verankerungselement der erfindungsgemäßen Implantatentfernungsvorrichtung dient, an welchem das Koppelorgan befestigt ist.

In einer bevorzugten Variante der Erfindung ist das Verankerungselement eine Verschlussschraube, die einen Außengewindeabschnitt aufweist, der mit einem am Implantat ausgebildeten Innengewindeabschnitt verschraubbar ist.

Das Koppelorgan kann eine langgestreckte Form aufweisen.

Das Koppelorgan kann beispielsweise ein Seil oder ein Draht sein.

Ferner kann vorgesehen sein, dass das Koppelorgan aus einer Mehrzahl von einzelnen Festigkeitsträgern aufgebaut ist. Diese Festigkeitsträger können miteinander verflochten oder verdreht sein, um das Koppelorgan zu bilden.

Das Koppelorgan kann ferner aus einem biokompatiblen Material hergestellt sein. Das Koppelorgan kann aus einem Nahtmaterial bestehen. Konkrete Materialbeispiele für das Koppelorgan sind Titan oder Stahl.

Des Weiteren kann vorgesehen sein, dass das Koppelorgan mit seinem einen Ende am Verankerungselement befestigt ist und an seinem anderen Ende den Handhabungsabschnitt aufweist.

Der Handhabungsabschnitt kann als Schlaufe, Schlinge oder Öse des Koppelorgans ausgebildet sein.

Die Erfindung betrifft außerdem ein Operationssystem mit einer Mehrzahl von Implantaten, insbesondere Marknägeln, und mit einer Mehrzahl von Vorrichtungen zur Implantatentfernung, wie sie vorstehend erläutert sind und deren Verankerungselement jeweils mit zumindest einem der Implantate verbindbar ist.

Weitere Ausführungsformen der Erfindung sind in den Unteransprüchen, der Beschreibung sowie der Zeichnung angegeben.

Die Erfindung wird im Folgenden beispielhaft unter Bezugnahme auf die Zeichnung beschrieben. Es zeigen:

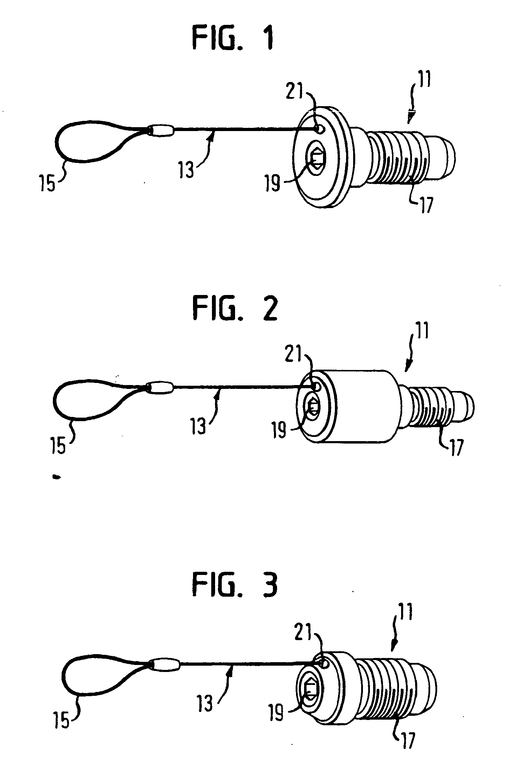

- Fig. 1-3: verschiedene Ausführungsformen einer erfindungsgemäßen Vorrichtung zur Implantatentfernung.

Die in Fig. 1-3 dargestellten Ausführungsformen einer erfindungsgemäßen Vorrichtung zur Implantatentfernung unterscheiden sich durch die Ausgestaltung des Verankerungselementes 11. Die Verankerungselemente 11 sind jeweils an das jeweilige Implantat (nicht dargestellt) angepasst.

In den dargestellten Ausführungsbeispielen handelt es sich bei den Verankerungselementen 11 jeweils um eine Verschlussschraube, die mit einem ein Innengewinde aufweisenden Befestigungsabschnitt des Implantats verschraubt werden kann, um den Befestigungsabschnitt beziehungsweise das Innengewinde vor dem Gewebe zu schützen.

Hierzu sind die Verankerungselemente 11 jeweils mit einem Außengewindeabschnitt 17 sowie einem Betätigungsabschnitt 19 für ein Werkzeug - hier einen Innensechskant - versehen.

Die konkrete Ausgestaltung des Verankerungselementes 11 der erfindungsgemäßen Implantatentfernungsvorrichtung ist grundsätzlich beliebig. Wichtig ist, dass eine sichere Verbindung zwischen dem Verankerungselement 11 und dem jeweiligen Implantat/möglich ist und am Verankerungselement 11 ein Koppelorgan 13 befestigt werden kann, auf das nachstehend näher eingegangen wird.

Bei dem Koppelelement 13 handelt es sich in den dargestellten Ausführungsformen jeweils um ein langgestrecktes, flexibles Organ in Form eines Seils, Drahtes oder Bandes. Während das eine Ende des Koppelorgans 13 an einer Verbindungsstelle 21 fest mit dem jeweiligen Verankerungselement 11 verbunden ist, beispielsweise durch Verschweißen, ist das freie Ende des Koppelorgans 13 als eine Schlinge oder Schlaufe 15 ausgebildet, die einen Handhabungsabschnitt der erfindungsgemäßen Implantatentfernungsvorrichtung bildet. Die Schlaufe bzw. Schlinge kann derart aus-geführt sein, dass sie sich auf Zug zuzieht. Alternativ bleibt die Größe der Schlaufe bzw. Schlinge auch bei Einwirkung von Zugkräften unverändert.

Der Handhabungsabschnitt 15 des Koppelorgans 13 muss nicht als Schlinge oder Schlaufe ausgebildet sein. Die Ausgestaltung des Handhabungsabschnitts 15 ist grundsätzlich beliebig und wird entsprechend dem jeweiligen Anwendungszweck gewählt.

Die Art und Weise der Befestigung des Koppelorgans 13 an der Verschlussschraube 11 ist grundsätzlich beliebig und wird insbesondere in Abhängigkeit von den für die Verschlussschraube 11 und das Koppelorgan 13 verwendeten Materialien gewählt. Die Art der Befestigung wird bevorzugt derart gewählt, dass auch relativ große Zugkräfte übertragen werden können, um das mit der Verschlussschraube 11 verbundene Implantat zumindest in Fällen, in denen das Implantat vergleichsweise locker sitzt, über das Koppelorgan 13 und die Verschlussschraube 11 aus dem Körper des Patienten herausziehen zu können.

Die flexible Ausgestaltung des Koppelorgans 13 gestattet es auf besonders einfache Weise, die Schlinge 15 an einer ausgewählten Stelle direkt unter der Haut zu platzieren. Zur Implantatentfernung kann dann an der entsprechenden Stelle die Haut eingeschnitten, das Koppelorgan 13 straff gespannt und das Gewebe bis zum Knochen entlang des Koppelorgans 13 stumpf präpariert werden, wenn ein Herausziehen des Implantats über das Koppelorgan 13, wie es vorstehend geschildert wurde, nicht möglich ist.

Durch die erfindungsgemäß geschaffene Möglichkeit zur Lokalisierung des Implantats mittels des insofern als Führung dienenden Seils 13 erübrigt sich eine ausgedehnte Suche nach dem Implantat im Gewebe, wodurch lediglich ein sehr kleiner Schnitt in der Haut benötigt wird, der nur das Erfassen der Schlinge 15 des Koppelorgans 13 zu ermöglichen braucht.

Folglich können dank der Erfindung die für den Patienten vorteilhaften minimalinvasiven Techniken auch bei der Implantatentfernung zum Einsatz kommen.

Bei den vorstehend beschriebenen Ausführungsformen nutzt die Erfindung in vorteilhafter Weise die ohnehin mit dem jeweils implantierten Marknagel verschraubte Verschlussschraube 11 als ein zum Auffinden bzw. zum Herausziehen des Marknagels dienendes Verankerungselement, an welchem das Koppelorgan 13 befestigt wird. Erfindungsgemäß ist es jedoch nicht zwingend, ein ohnehin vorhandenes Bauteil gleichzeitig als Verankerungselement zu nutzen, sondern es ist auch möglich, gezielt für den Zweck der Implantatentfernung hergestellte Verankerungselemente zu verwenden, die derart ausgebildet sind, dass sie in geeigneter Weise mit dem Implantat verbunden werden können.

### Bezugszeichenliste

- 11: Verankerungselement
- 13: Koppelorgan
- 15: Handhabungsabschnitt
- 17: Außengewindeabschnitt
- 19: Betätigungsabschnitt
- 21: Verbindungsstelle

## Patentansprüche

1. Vorrichtung zur Implantatentfernung mit einem Verankerungselement (11), das mit dem Implantat verbindbar ist, und mit einem Koppelorgan (13), das am Verankerungselement (11) befestigt ist, **dadurch gekennzeichnet, dass** das Koppelorgan (13) flexibel ausgeführt ist, und eine Länge derart aufweist, dass ein Handhabungsabschnitt (15) des Koppelorgans (13) an einer vom mit dem Implantat verbundenen Verankerungselement (11) entfernt gelegenen Stelle im Körper des Patienten platzierbar ist.

2. Vorrichtung nach Anspruch 1,
**dadurch gekennzeichnet,**
**dass** das Verankerungselement (11) mit dem Implantat verschraubbar ist.

3. Vorrichtung nach Anspruch 1 oder 2,
**dadurch gekennzeichnet,**
**dass** das Verankerungselement (11) ein Abschlusselement zum Schutz eines zum Einbringen des Implantats verwendbaren Befestigungsabschnitts, insbesondere eines Gewindeabschnitts, des Implantats ist.

4. Vorrichtung nach einem der vorhergehenden Ansprüche,
**dadurch gekennzeichnet,**
**dass** das Verankerungselement (11) eine Verschlussschraube ist und einen Außengewindeabschnitt (17) aufweist, der mit einem am Implantat ausgebildeten Innengewindeabschnitt verschraubbar ist.

5. Vorrichtung nach einem der vorhergehenden Ansprüche,
**dadurch gekennzeichnet,**
**dass** das Koppelorgan (13) eine langgestreckte Form aufweist.

6. Vorrichtung nach einem der vorhergehenden Ansprüche,
**dadurch gekennzeichnet,**
**dass** das Koppelorgan (13) ein Seil ist.

7. Vorrichtung nach einem der vorhergehenden Ansprüche,
**dadurch gekennzeichnet,**
**dass** das Koppelorgan (13) aus einer Mehrzahl von einzelnen Festigkeitsträgern aufgebaut ist.

8. Vorrichtung nach einem der vorhergehenden Ansprüche,
**dadurch gekennzeichnet,**
**dass** das Koppelorgan (13) ein Draht ist.

9. Vorrichtung nach einem der vorhergehenden Ansprüche,
**dadurch gekennzeichnet,**
**dass** das Koppelorgan (13) aus einem biokompatiblen Material hergestellt ist.

10. Vorrichtung nach einem der vorhergehenden Ansprüche,
**dadurch gekennzeichnet,**
**dass** das Koppelorgan (13) aus einem Nahtmaterial hergestellt ist.

11. Vorrichtung nach einem der vorhergehenden Ansprüche,
**dadurch gekennzeichnet,**
**dass** das Koppelorgan (13) aus Titan oder Stahl hergestellt ist.

12. Vorrichtung nach einem der vorhergehenden Ansprüche,
**dadurch gekennzeichnet,**
**dass** das Koppelorgan (13) mit seinem einen Ende am Verankerungselement (11) befestigt ist und an seinem anderen Ende den Handhabungsabschnitt (15) aufweist.

13. Vorrichtung nach Anspruch 12,
**dadurch gekennzeichnet,**
**dass** der Handhabungsabschnitt (15) als Schlaufe, Schlinge oder Öse des Koppelorgans (13) ausgebildet ist.

14. Vorrichtung nach einem der vorhergehenden Ansprüche,
**dadurch gekennzeichnet,**
**dass** die Länge des Koppelorgans (13) derart bemessen ist, dass der Handhabungsabschnitt (15) direkt unter der Haut des Patienten platzierbar ist.

15. Vorrichtung nach einem der vorhergehenden Ansprüche,
**dadurch gekennzeichnet,**
**dass** es zur Entfernung von Marknägeln ausgebildet ist.

16. Operationssystem mit einer Mehrzahl von Implantaten, insbesondere Marknägeln, und mit einer Mehrzahl von Implantatentfernungsvorrichtungen nach einem der vorhergehenden Ansprüche, deren Verankerungselement jeweils mit zumindest einem der Implantate verbindbar ist.

## Claims

1. An apparatus for implant removal having an anchoring element (11) which can be connected to the implant and having a coupling member (13) which is fastened to the anchoring element (11),
**characterised in that** the coupling member (13) is flexible and has a length such that a handling section (15) of the coupling member (13) can be placed at a position in the patient's body disposed remotely from the anchoring element (11) connected to the implant.

2. An apparatus in accordance with claim 1, **characterised in that** the anchoring element (11) can be screwed to the implant.

3. An apparatus in accordance with claim 1 or claim 2, **characterised in that** the anchoring element (11) is a terminal element for the protection of a fastening section, in particular of a thread section, of the implant usable for the insertion of the implant.

4. An apparatus in accordance with any one of the preceding claims, **characterised in that** the anchoring element (11) is a plug screw and has an externally threaded section (17) which can be screwed to the internally threaded section formed at the implant.

5. An apparatus in accordance with any one of the preceding claims, **characterised in that** the coupling member (13) has an elongate shape.

6. An apparatus in accordance with any one of the preceding claims, **characterised in that** the coupling member (13) is a cable.

7. An apparatus in accordance with any one of the preceding claims, **characterised in that** the coupling member (13) is made up of a plurality of individual load carriers.

8. An apparatus in accordance with any one of the preceding claims, **characterised in that** the coupling member (13) is a wire.

9. An apparatus in accordance with any one of the preceding claims, **characterised in that** the coupling member (13) is manufactured from a biocompatible material.

10. An apparatus in accordance with any one of the preceding claims, **characterised in that** the coupling member (13) is made of a suture material.

11. An apparatus in accordance with any one of the preceding claims, **characterised in that** the coupling member (13) is manufactured from titanium or steel.

12. An apparatus in accordance with any one of the preceding claims, **characterised in that** the coupling member (13) is fastened at its one end to the anchoring element (11) and has the handling section (15) at its other end.

13. An apparatus in accordance with claim 12, **characterised in that** the handling section (15) is formed as a loop, a sling or an eye of the coupling member (13).

14. An apparatus in accordance with any one of the preceding claims, **characterised in that** the length of the coupling member (13) is dimensioned such that the handling section (15) can be placed directly under the skin of the patient.

15. An apparatus in accordance with any one of the preceding claims, **characterised in that** it is made for the removal of medullary nails.

16. An operating system having a plurality of implants, in particular of medullary nails, and having a plurality of implant removal apparatuses in accordance with any one of the preceding claims whose anchoring element can in each case be connected to at least one of the implants.

## Revendications

1. Instrument servant à l'enlèvement d'implants, comportant un élément d'ancrage (11) susceptible d'être relié à l'implant, et un organe d'accouplement (13) fixé sur l'élément d'ancrage (11),
**caractérisé en ce que**
l'organe d'accouplement (13) est réalisé flexible et présente une longueur telle qu'un tronçon de maniement (15) de l'organe d'accouplement (13) est susceptible d'être placé dans le corps du patient à un emplacement éloigné de l'élément d'ancrage (11) relié à l'implant.

2. Instrument selon la revendication 1,
**caractérisé en ce que**
l'élément d'ancrage (11) est susceptible d'être vissé sur l'implant.

3. Instrument selon l'une ou l'autre des revendications 1 et 2,
**caractérisé en ce que**
l'élément d'ancrage (11) est un élément de terminaison pour protéger un tronçon de fixation, en particulier un tronçon à pas de vis, de l'implant, utilisable pour introduire l'implant.

4. Instrument selon l'une des revendications précédentes,
**caractérisé en ce que**
l'élément d'ancrage (11) est une vis d'obturation et comprend un tronçon fileté (17) qui est susceptible d'être vissé dans un tronçon taraudé ménagé dans l'implant.

5. Instrument selon l'une des revendications précédentes,
**caractérisé en ce que**
l'organe d'accouplement (13) présente une forme allongée.

6. Instrument selon l'une des revendications précédentes,
**caractérisé en ce que**
l'organe d'accouplement (13) est un câble.

7. Instrument selon l'une des revendications précédentes,
**caractérisé en ce que**
l'organe d'accouplement (13) est constitué à partir d'une pluralité de supports solides individuels.

8. Instrument selon l'une des revendications précédentes,
**caractérisé en ce que**
l'organe d'accouplement (13) est un fil.

9. Instrument selon l'une des revendications précédentes,
**caractérisé en ce que**
l'organe d'accouplement (13) est réalisé à partir d'un matériau biocompatible.

10. Instrument selon l'une des revendications précédentes,
**caractérisé en ce que**
l'organe d'accouplement (13) est réalisé à partir d'un matériau de suture.

11. Instrument selon l'une des revendications précédentes,
**caractérisé en ce que**
l'organe d'accouplement (13) est réalisé en titane ou en acier.

12. Instrument selon l'une des revendications précédentes,
**caractérisé en ce que**
l'organe d'accouplement (13) est fixé par l'une de ses extrémités sur l'élément d'ancrage (11) et comprend à son autre extrémité le tronçon de maniement (15).

13. Instrument selon la revendication 12,
**caractérisé en ce que**
le tronçon de maniement (15) est réalisé sous forme de boucle, d'anneau ou d'oeillet de l'organe d'accouplement (13).

14. Instrument selon l'une des revendications précédentes,
**caractérisé en ce que**
la longueur de l'organe d'accouplement (13) est dimensionnée de telle sorte que le tronçon de maniement (15) est susceptible d'être placé directement sous la peau du patient.

15. Instrument selon l'une des revendications précédentes,
**caractérisé en ce que**
il est réalisé pour enlever des clous hors du canal médullaire.

16. Système d'opération comportant une pluralité d'implants, en particulier de clous pour canal médullaire, et comportant une pluralité d'instruments d'enlèvement d'implants selon l'une des revendications précédentes, dont l'élément d'ancrage est susceptible d'être relié chacun à l'un au moins des implants.
